# EUROPEAN PATENT APPLICATION

(11) **EP 1 683 521 A1**
(43) Date of publication of application: **26.07.2006**
(21) Application number: 05290137.8
(22) Date of filing: 21.01.2005
(51) Int. Cl.: A61K 31/404, A61K 31/4045, A61P 31/00, A61P 31/04

(54) **Peptide deformylase inhibitors, their use, and pharmaceutical compositions containing the same**

(71) Applicant: CENTRE NATIONAL DE LA RECHERCHE SCIENTIFIQUE (CNRS), 75794 Paris Cedex 16 (FR); UNIVERSITE RENE DESCARTES (PARIS V), F-75270 Paris Cédex 06 (FR)
(72) Inventor: Meinnel, Thierry, 92340 Bourg-la-Reine (FR); Artaud, Isabelle, 92350 Le Plessis Robinson (FR); Boularot, Adrien, 94410 Saint-Maurice (FR); Dardel, Frédéric, 75009 Paris (FR); Giglione-Meinnel, Carmela, 92340 Bourg-La-Reine (FR); Alves De Sousa, Rodolphe, 94200 Ivry Sur Seine (FR); Larue, Valery, 75013 Paris (FR)
(74) Representative: Grosset-Fournier, Chantal Catherine

(57) **Abstract**

The present invention relates to the use of indole derivatives or of pharmaceutically acceptable salts thereof, for the manufacture of medicaments intended for the prevention or the treatment of bacterial infections or protozoan infections, or for the manufacture of herbicides.

### SEQUENCE LISTING

<110> CENTRE NATIONAL DE LA RECHERCHE SCIENTIFIQUE UNIVERSITE PARIS 5
<120> PEPTIDE DEFORMYLASE INHIBITORS, THEIR USE, AND PHARMACEUTICAL COMPOSITIONS CONTAINING THE SAME
<130> IOB 04 DM CNR INDO
<160> 2
<170> PatentIn version 3.1
<210> 1
   <211> 169
   <212> PRT
   <213> Escherichia coli
<400> 1
   Met Ser Val Leu Gln Val Leu His Ile Pro Asp Glu Arg Leu Arg Lys
   1               5                   10                  15
   Val Ala Lys Pro Val Glu Glu Val Asn Ala Glu Ile Gln Arg Ile Val
               20                  25                  30
   Asp Asp Met Phe Glu Thr Met Tyr Ala Glu Glu Gly Ile Gly Leu Ala
           35                  40                  45
   Ala Thr Gln Val Asp Ile His Gln Arg Ile Ile Val Ile Asp Val Ser
       50                  55                  60
   Glu Asn Arg Asp Glu Arg Leu Val Leu Ile Asn Pro Glu Leu Leu Glu
   65                  70                  75                  80
   Lys Ser Gly Glu Thr Gly Ile Glu Glu Gly Cys Leu Ser Ile Pro Glu
                   85                  90                  95
   Gln Arg Ala Leu Val Pro Arg Ala Glu Lys Val Lys Ile Arg Ala Leu
               100                 105                 110
   Asp Arg Asp Gly Lys Pro Phe Glu Leu Glu Ala Asp Gly Leu Leu Ala
           115                 120                 125
   Ile Cys Ile Gln His Glu Met Asp His Leu Val Gly Lys Leu Phe Met
       130                 135                 140
   Asp Tyr Leu Ser Pro Leu Lys Gln Gln Arg Ile Arg Gln Lys Val Glu
   145                 150                 155                 160
   Lys Leu Asp Arg Leu Lys Ala Arg Ala
                   165
<210> 2
   <211> 184
   <212> PRT
   <213> Bacillus stearothermophilus
<400> 2
   Met Ile Thr Met Lys Asp Ile Ile Lys Glu Gly His Pro Thr Leu Arg
   1               5                   10                  15
   Lys Val Ala Glu Pro Val Pro Leu Pro Pro Ser Glu Glu Asp Lys Arg
               20                  25                  30
   Ile Leu Gln Ser Leu Leu Asp Tyr Val Lys Met Ser Gln Asp Pro Glu
           35                  40                  45
   Leu Ala Ala Lys Tyr Gly Leu Arg Pro Gly Ile Gly Leu Ala Ala Pro
       50                  55                  60
   Gln Ile Asn Val Ser Lys Arg Met Ile Ala Val His Val Thr Asp Glu
   65                  70                  75                  80
   Asn Gly Thr Leu Tyr Ser Tyr Ala Leu Phe Asn Pro Lys Ile Val Ser
                   85                  90                  95
   His Ser Val Gln Gln Cys Tyr Leu Thr Thr Gly Glu Gly Cys Leu Ser
               100                 105                 110
   Val Asp Arg Asp Val Pro Gly Tyr Val Leu Arg Tyr Ala Arg Ile Thr
           115                 120                 125
   Val Thr Gly Thr Thr Leu Asp Gly Glu Glu Val Thr Leu Arg Leu Lys
       130                 135                 140
   Gly Leu Pro Ala Ile Val Phe Gln His Glu Ile Asp His Leu Asn Gly
   145                 150                 155                 160
   Ile Met Phe Tyr Asp Arg Ile Asn Pro Ala Asp Pro Phe Gln Val Pro
                   165                 170                 175
   Asp Gly Ala Ile Pro Ile Gly Arg
               180

## Description

The present invention relates to peptide deformylase inhibitors, to pharmaceutical compositions containing them, and to their use as herbicides or for treating bacterial or parasitic infections.

Peptide deformylase (PDF; EC 3.5.1.88) is an essential enzyme activity found in all Gram-positive and Gram-negative bacteria. Its cellular role is to remove the N-formyl group from all nascent proteins. This allows the action of methionine aminopeptidase, another essential bacterial enzyme responsible for N-terminal methionine excision (for a review see Giglione *et al*., 2004). PDF is the natural target of actinonin, a natural antibiotic substance produced by a *Streptomyces* sp. (Gordon *et al*., 1962; Chen *et al*., 2000). Although less potent, other natural products inhibiting PDF also produced by *Streptomyces* sp. were described (Chu *et al*., 2001).

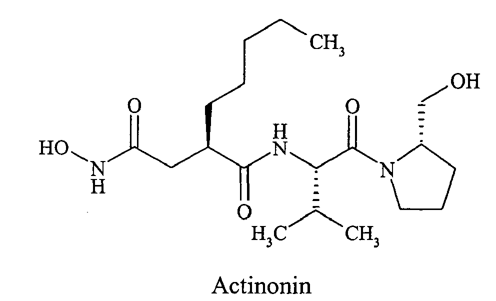

Based both on sequence and structural analysis, two bacterial PDF types - PDF1 and PDF2 - have been distinguished (Giglione *et al*., 2000a; Guilloteau *et al.,* 2002). PDF2 are found only in Gram-positive bacteria. Because high-resolution three-dimensional (3D) structure and in depth enzymatic analyses are available, *Escherichia coli* PDF (PDF1B) and *Bacillus stearothermophilus* PDF2 (PDF2) were chosen as the representative of either PDF class (Guilloteau *et al*., 2002; *Ragusa et al*., 1998). Bacteria can have one or several functional genes encoding a PDF. For instance, *Escherichia coli* has one PDF1 gene encoded by the *def* cistron, major pathogens such as *Staphylo- and Streptococci* spp. have only one PDF2 and *Bacillus* spp. have two PDF genes, the *def* gene encoding a PDF1 and *ykrB* a PDF2. In *B. subilis*, PDF2 is the major PDF *(Haas et al*., 2001).

For the past decade, PDF has been selected as a target of choice for the selection of new antibiotics using state-of-the-art drug discovery methods (Giglione *et al.,* 2000a; Meinnel, 2000; Giglione & Meinnel, 2001). During the past 4 years, a number of PDF inhibitors (PDFI) mimicking actinonin were described and several of them recently entered clinical trial (for a review see Boularot *et al*., 2004). Hence, PDFI are the first success for antibacterial drug discovery arising from genomics strategies (Miesel *et al.,* 2003).

However, although initially thought to be a unique feature of the eubacteria (Mazel *et al*., 1994), functional PDF sequences have been detected most recently in most eukaryote genomes (Giglione *et al*., 2000b; Bracchi-Ricard *et al*., 2001). These PDF are targeted to the organelles, the mitochondrion and the plastid of plants (chloroplasts) and apicomplexan parasites (apicoplasts) such as the agent of malaria, *Plasmodium falciparum*. Furthermore, PDF homologs have also been evidenced in kinetoplastid protozoans (Meinnel, 2000).

Based on sequence or biochemical analysis and site-directed mutagenesis data, all mitochondrial PDFs were shown to belong to a unique sub-class of type 1 PDFs (PDF1A) and their active site showed a number of singularities (Giglione *et al*., 2000b; *Serero et al*., *2001; Serero et al*., 2003). Plastid PDFs are strongly related to bacterial PDF1s and are classified as PDF1B (Giglione *et al*., 2004).

As actinonin shows inhibitory effects against both *P. falciparum* and plant PDF in *vitro* and in *vivo,* this discovery first suggested that PDFI active against bacterial PDF could be used as antiparasitic agents and even as herbicides (Meinnel, 2000; Giglione & Meinnel, 2001; *Wiesner et al*., 2001; Serero *et al*., 2001; Serero *et al*., 2001; Dirk *et al*., 2001).

Nevertheless, the occurrence of a PDF1A in the human mitochondria (HsPDF1) raised a number of issues regarding the toxicity of the above-described PDFI, especially as actinonin (i) blocks efficiently in *vitro* HsPDF1 activity and (ii) exerts cytotoxic activity in a range of concentrations (2-3 µg/ml) similar to the minimal inhibitory concentration (MIC) of the best PDFI on the most sensitive Gram-positive bacteria (Lee *et al*., 2003; *Serero et al*., 2003; *Xu et al*., 1998; *Grujic et al*., 2002, *Lee et al* 2004).

Although the role of mitochondrial PDF remains unclear and the mitochondrial membrane is generally considered to be very impermeable to most compounds, the precautionary principle imposes that great caution should now be given to avoid any cross-inhibition of PDFI between PDF1A and PDF1B or PDF2. In other words, this means that new-generation PDFI should not show any inhibitory effect on mitochondrial PDF (PDF1A), including at least HsPDF.

Thus, an object of the present invention is to provide new compounds which are liable to potently inhibit peptide deformylases from bacteria, plant plastids, and protozoans such as apicocomplexan and kinetoplastid protists, but not from mitochondria, and in particular from mammalian mitochondria.

Another object of the present invention relates to the use of said compounds for treating individuals with a bacterial or a parasitic infection, or as herbicides.

The present invention follows on from the unexpected finding by the Inventors that indolic derivatives could potently inhibit bacterial peptide deformylases while being essentially inactive on human mitochondrial peptide deformylase.

The present invention relates to the use of compounds having:
a) the following general formula (I): 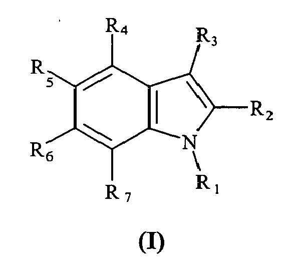 wherein:
   - independently from each other, R₂ and R₃ represent H, methyl, ethyl, propyl, or a metal chelating group from 2 to 20 atoms comprising from 1 to 5 heteroatoms;
   - independently from one another, R₄, R₅ and R₆ represent H, a halogen, or a group comprising from 1 to 10 carbon atoms;
   - independently from each other R₁ and R₇ represents H, or a group comprising from 1 to 50 carbon atoms;
      provided that at least one of R₂ and R₃ represents a metal chelating group as defined above and at least one of R₄, R₅ and R₆ does not represent H; and
b) an IC₅₀ lower than about 1 µM with respect to the *Escherichia coli* nickel-bound peptide deformylase (SEQ ID NO: 1) and/or to the *Bacillus stearothermophilus* nickel-bound peptide deformylase (SEQ ID NO: 2),
or of pharmaceutically acceptable salts thereof,
for the manufacture of medicaments intended for the prevention or the treatment of bacterial or protozoan infections, or for the manufacture of herbicides.

A "metal chelating group" as mentioned above relates to a group which is liable to form coordination complexes with metal atoms or metallic ions (Whittaker *et al.,* 1999 ; *Boularot et al*., 2004). Thus a "metal chelating group" relates, in particular, to a group bearing one or several free electronic doublets.

In a preferred embodiment, the invention relates to the use of compounds of the general formula (I) as defined above, wherein, independently from each other, R₂ and R₃ represent H, methyl, ethyl, propyl, or a metal chelating group of the following formula:

-(CH₂)ₙ-X

wherein:
- n is an integer from 0 to 3, and
- X represents a group selected from the list comprising -NH₂ (amine), -NHOH (hydroxylamine), =NOH (oxime), -NHCN (cyanamide), -NH-C(NOH)NH₂ (hydroxyguanidine), -SCN (thiocyanate), -SH (thiol), -B(OH)₂ (boronic acid),
- COCH₂OH (hydroxymethylketone), -COCH₂SH (thiomethylketone), -CONHOH (hydroxamic acid), -SO₂NHOH (sulfohydroxamic acid), -NO-N=O (nonoate), -NOH-COH (reverse hydroxamic acid).

In another preferred embodiment, the invention also relates to the use of compounds of the general formula (I) as defined above, wherein, independently from each other, R₂ and R₃ represent H, methyl, ethyl, propyl, or a metal chelating group of the following formula:

-(CH₂)ₙ-Y-Z

wherein:
- n is an integer from 0 to 3, and
- Y represents a group selected from the list comprising -O-, -NH-, -CHOH-, -CHF-, or 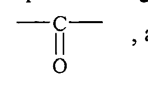 and
- Z represents a group selected from the list comprising -COOH, -CONHOH, or -COCH₂SH.

In another preferred embodiment, the invention relates to the use of compounds of the general formula (I) as defined above, wherein, independently from each other, R₄, R₅ and R₆ represent H, a halogen, an alkyl, an alkoxy, a thioalkyl, a thioaryl, an acetyl, an alkoxycarbonyl, an aryl, an aryloxy, or an aryloxycarbonyl group, if adequate substituted by a hydroxyl, a thiol, a thioalkyl, a thioaryl, or an amino group.

According to a preferred embodiment, the invention also relates to the use of compounds of the general formula (I) as defined above, wherein R₁ represents
- H, or
- a peptide sequence, or
- an alkyl, an alkoxy, an alkoxycarbonyl, an aryl, an aryloxy, a thioalkyl, a thioaryl, an acetyl, an aryloxycarbonyl, an alkoxycarbonylalkyl, or an aryloxycarbonylalkyl group, if adequate substituted by a pyridine, a morpholine, a thiomorpholoine, a piperidine, a piperazine, a hydroxyl, a thiol, a thioalkyl, a thioaryl or an amino group.

In another preferred embodiment, the invention relates to the use of compounds of the general formula (I) as defined above, wherein R₇ represents H or an alkyl group comprising from 1 to 10 carbon atoms.

According to a particularly preferred embodiment, the present invention relates to the use of compounds of the general formula (I) as defined above, wherein:
- one of R₂ and R₃ represents H or CH₃, and
- two of R₄, R₅ and R₆ represent H.

According to another particularly preferred embodiment, the present invention relates to the use of compounds of the general formula (I) as defined above, wherein:
- R₂ represents H or CH₃, and
- R₄ and R₆ represent H, and
- R₇ represents H.

In another particularly preferred embodiment, the present invention relates to the compounds of the general formula (I) as defined above, wherein the metal chelating group comprises or consists of a hydroxamic acid group.

In a particularly preferred embodiment, the present invention relates to the use of compounds of the general formula (I) as defined above, characterized in that said compounds correspond to the following formulae:

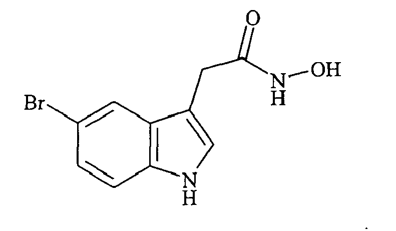

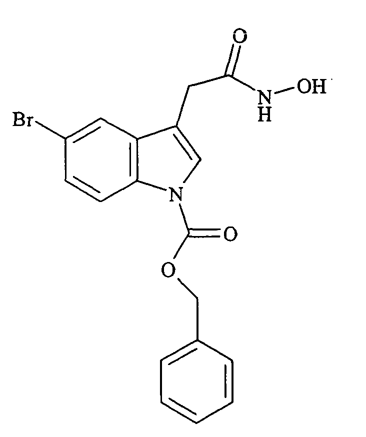

Peptide deformylase (PDF) is a metallo-enzyme (*i.e*. its prosthetic group is a metallic ion). Thus, as intended herein, the expression "nickel-bound peptide deformylase" relates to an enzyme which contains essentially only nickel as its metallic ion. For instance, such an enzyme can be obtained by purifying it in the presence of nickel as described in Ragusa *et al*. (1998).

The expression IC₅₀ relates to the concentration of compounds of the general formula (I) which is necessary to inhibit 50% of the activity the *Escherichia coli* nickel-bound peptide deformylase (SEQ ID NO: 1) (EMBL/GenBank accession number P27251) and/or to the *Bacillus stearothermophilus* nickel-bound peptide deformylase (SEQ ID NO: 2) (EMBL/GenBank accession number 031410).

The IC₅₀ for peptide deformylase can be measured by following the general method described in *Serero et al.* (2003).

Briefly, prior to kinetic analysis (as described in Lazennec & Meinnel, 1997), inhibitors are incubated with the enzyme studied at a set final concentration for 15 minutes at 25°C. Kinetic assays are started by adding a small volume of the substrate. The substrate is 1 mM Formyl-Met-Ala-Ser. The kinetic analysis is performed in the presence of an enzyme concentration giving a deformylation rate of 0.5 µM/s in the absence of inhibitor. The IC₅₀ value corresponds to a concentration giving 50% inhibition.

Advantageously, the compounds of general formula (I) are liable to inhibit bacterial PDF (*i*.*e*. PDF1 and PDF2), as well as chloroplastic, apicoplastic and kinetoplastic PDF, but not the mitochondrial PDF (*i*.*e*. PDF1A).

In another particularly preferred embodiment, the present invention relates to the use of compounds of the general formula (I) as defined above, wherein the protozoans are chosen among the apicoplast or kinetoplast bearing protozoans, and in particular are selected from the group comprising the protozoans of the foiiowing genii: *Plasmodium, Toxoplasma, Sarcocystis, Cryptosporidium, Eimera, Theileria, Babesia, Trypanosoma, Leishmania.*

Advantageously, the compounds of formula (I) target and inhibit the apicoplastic or the kinetoplastic PDF and thus respectively impair the function of the apicoplast and of the kinetoplast, which inhibits the growth of protozoan parasites and/or kills them.

In yet another particularly preferred embodiment, the present invention relates to the use of compounds of the general formula (I) as defined above, wherein the bacteria are selected from the group comprising the bacteria of the following genii: *Staphylococcus, Streptococcus, Bacillus, Enterococcus, Pseudomonas, Acinetobacter, Enterobacter, Haemophilus*.

Advantageously, the compounds of general formula (I) target and inhibit both the bacterial PDF1 and PDF2, which makes them broad-spectrum antibiotics which can be used both against Gram-negative and Gram-positive bacteria.

The present invention also relates to a pharmaceutical composition, characterized in that it comprises at least one compound of formula (I) as defined above, or a pharmaceutically acceptable salt thereof, as active substance, in association with a pharmaceutically acceptable vehicle.

In a preferred embodiment, the above defined pharmaceutical composition is suitable for the administration to an individual of a unit dose of about 250 mg to about 5000 mg of the compound of formula (I).

In another preferred embodiment, the above defined pharmaceutical composition is suitable for the administration to an individual of a daily dose of about 250 mg to about 5000 mg of the compound of formula (I).

In another preferred embodiment, the above defined pharmaceutical composition is administered by oral, intra-venous, or intra-peritoneal route.

The present invention also relates to an herbicide composition, characterized in that it comprises at least one compound of formula (I) as defined above as active substance.

In a preferred embodiment, the above defined herbicidal composition is suitable for the administration to a plant of a dose of about 1 mg/l to about 1000 mg/l, in particular of about 10 mg/l to about 100 mg/l, of the compound of formula (I).

Advantageously, the compounds of formula (I) target and inhibit the chloroplastic PDF, which either kills or severely impairs the growth of chloroplasts holding organisms, such as plants or algae for example.

The present invention also relates to a compound of the general formula (I), such as defined above.

In a preferred embodiment, the invention relates to the compounds of the general formula (I) as defined above, wherein, independently from each other, R₂ and R₃ represent H, methyl, ethyl, propyl, or a metal chelating group of the following formula:

-(CH₂)ₙ-X

wherein:
- n is an integer from 0 to 3, and
- X represents a group selected from the list comprising -NH₂ (amine), -NHOH (hydroxylamine), =NOH (oxime), -NHCN (cyanamide), -NH-C(NOH)NH₂ (hydroxyguanidine), -SCN (thiocyanate), -SH (thiol), -B(OH)₂ (boronic acid),
- COCH₂OH (hydroxymethylketone), -COCH₂SH (thiomethylketone), -CONHOH (hydroxamic acid), -SO₂NHOH (sulfohydroxamic acid), -NO-N=O (nonoate), or
- NOH-COH (reverse hydroxamic acid).

In another preferred embodiment, the invention also relates to the compounds of the general formula (I) as defined above, wherein, independently from each other, R₂ and R₃ represent H, methyl, ethyl, propyl, or a metal chelating group of the following formula:

-(CH₂)ₙ-Y-Z

wherein:
- n is an integer from 0 to 3, and
- Y represents a group selected from the list comprising -O-, -NH-, -CHOH-, -CHF-, or 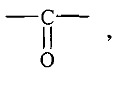 and
- Z represents a group selected from the list comprising -COOH, -CONHOH, or -COCH₂SH.

In another preferred embodiment, the invention relates to the compounds of the general formula (I) as defined above, wherein, independently from each other, R₄, R₅ and R₆ represent H, a halogen, an alkyl, an alkoxy, a thioalkyl, a thioaryl, an acetyl, an alkoxycarbonyl, an aryl, an aryloxy, or an aryloxycarbonyl group, if adequate substituted by a hydroxyl, a thiol, a thioalkyl, a thioaryl or an amino group.

According to a preferred embodiment, the invention also relates to the compounds of the general formula (I) as defined above, wherein R₁ represents
- H, or
- a peptide sequence, or
- an alkyl, an alkoxy, an alkoxycarbonyl, an aryl, an aryloxy, a thioalkyl, a thioaryl, an acetyl, an aryloxycarbonyl, an alkoxycarbonylalkyl, or an aryloxycarbonylalkyl group, if adequate substituted by a pyridine, a morpholine, a thiomorpholoine, a piperidine, a piperazine, a hydroxyl, a thiol, a thioalkyl, a thioaryl, or an amino group.

In another preferred embodiment, the invention relates to the compounds of the general formula (I) as defined above, wherein R₇ represents H or an alkyl group comprising from 1 to 10 carbon atoms.

According to a particularly preferred embodiment, the present invention relates to the compounds of the general formula (I) as defined above, wherein:
- one of R₂ and R₃ represents H or CH₃, and
- two of R₄, R₅ and R₆ represent H.

According to another particularly preferred embodiment, the present invention relates to the compounds of the general formula (I) as defined above, wherein:
- R₂ represents H or CH₃, and
- R₄ and R₆ represent H, and
- R₇ represents H.

In another particularly preferred embodiment, the present invention relates to the compounds of the general formula (I) as defined above, wherein the metal chelating group comprises or consists of a hydroxamic acid group.

In a particularly preferred embodiment, the present invention relates to the compounds of the general formula (I) as defined above, characterized in that said compounds correspond to the following formulae:

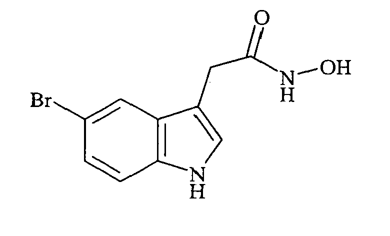

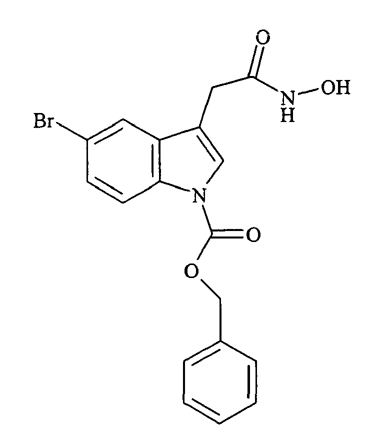

The present invention also relates to a method for screening compounds comprising an indole structure intended for the prevention or the treatment of bacterial or protozoan infections, or for their use as herbicides, characterized in that it comprises the steps of contacting a peptide deformylase enzyme with the compounds to screen and measuring the inhibiting activity of said compounds towards said peptide deformylase enzyme.

As intended herein, "compounds comprising an indole structure" are compounds which carry the following structure: 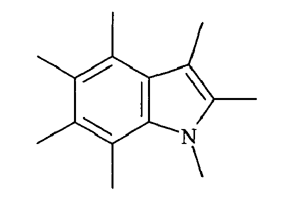 wherein the free bonds are either linked to H or to any chemical group.

In a preferred embodiment of the above defined screening method, the inhibiting activity of the compounds to screen towards the peptide deformylase enzyme is evaluated by measuring the IC₅₀ of said compounds with respect to said peptide deformylase enzyme.

The IC₅₀ can be measured according to the above described method.

In another preferred embodiment of the above defined screening method, the compounds to screen for which the IC₅₀ is lower than about 1 µM are selected.

In another preferred embodiment of the above defined screening method, the compounds to screen respond to formula (I) as defined above.

In another preferred embodiment of the above defined screening method, the peptide deformylase enzyme is *Escherichia coli* nickel-bound peptide deformylase (SEQ ID NO: 1) and/or *Bacilus stearothermophilus* nickel-bound peptide deformylase (SEQ ID NO: 2).

In a particularly preferred embodiment of the above defined screening method, it is further tested that the compounds to screen have essentially no inhibiting properties towards mitochondrial peptide deformylases, in particular human mitochondrial peptide deformylases.

The synthesis of compounds having the general formula (I) can be carried out according to the following procedures or as described in the examples.

Amine, cyanamide and hydroxyguanidine indolic derivatives can be prepared as depicted in the following Scheme 1.

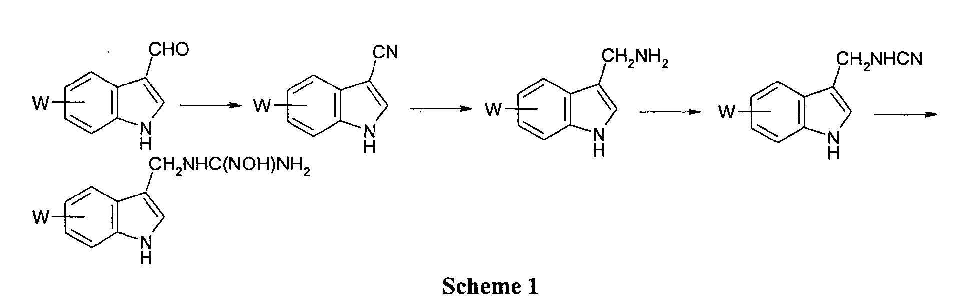

W represents a halogen, an alkyl, an aryl, an alkoxy, an aryloxy, a thioalkyl, or a thioaryl group.

Briefly, indole-3-carboxaldehyde prepared from the corresponding indole by the Vielsmeier-Haack reaction is transformed into indole-3-carbonitrile by treatment with diammonium hydrogen phosphate, 1-nitropropane and acetic acid (Jiang *et al.,* 2000). The nitrile is reduced into the amine with CoCl₂ / NaBH₄ (Leclerc *et al.,* 1998). The cyanamide is obtained by reaction of the amine with BrCN and converted into the hydroxyguanidine by reaction with hydroxylamine hydrochloride in the presence of sodium acetate (Lefevre-Groboillot *et al.,* 2001).

Hydroxamic acid, hydroxymethylketone and thiomethylketone indolic derivatives can be prepared as depicted in the following Scheme 2.

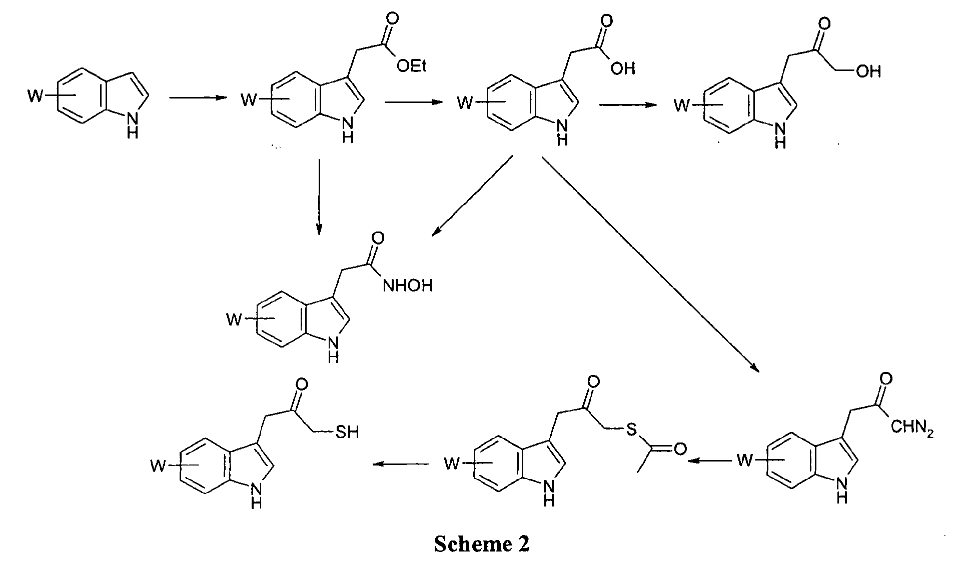

W represents an alkyl, an aryl, an alkoxy, an aryloxy or a thioaryl group.

Briefly, an acetic acid group is introduced into the 3-indole position by reacting the zinc salt with 2-bromoacetic acid ethyl esters followed by ester hydrolysis (Dillard et al., 1996). The hydroxymethylketone is prepared following the procedure described by Wissner *(Wissner et al.,* 1979) by reaction of the (indol-3-yl)acetyl chloride with tris(trimethylsilyloxy)ethylene, without any Lewis acid catalyst, at room temperature (r.t.), followed by hydrolysis decarboxylation of the intermediate at 80°C. Thioacetyl derivatives are prepared in five steps starting from the acids which are converted into the α-diazoketones by reaction of the acylchloride with diazomethane (Salim & Capretta, 2000). Treatment with an etheral HCl(g) solution affords the chloromethylketones. Displacement of the chlorides with potassium thioacetate at r.t , in DMF yielded the corresponding thioesters which can be deprotected by hydrolysis under strictly anaerobic conditions with Na₂CO₃ in methanol followed by acidification with an etheral HCl(g) solution.

A general procedure for the preparation of hydroxamic acid indolic derivatives from the corresponding acid indolic derivatives is as follows.

Some of the (indol-3-yl)acetic acid derivatives are commercially available, so a general synthetic procedure starting from the acids is described hereafter, even though the direct reaction of hydroxylamine with the ester is possible.

Briefly, to a 10 ml DMF solution of the indolacetic acid derivative (0.3 mmol) are added 1-hydroxybenzotriazole (1.1 equiv., m = 44 mg), 1-(3-dimethylaminopropyl)-3-ethylcarbodiimide hydrochloride (1.1 equiv., m = 63 mg) and N-methylmorpholine (1.1 equiv., 36 µL). This solution is stirred for one hour at r.t. After adding hydroxylamine hydrochloride (1.1 equiv., m = 23 mg) the solution is left under stirring overnight. Then DMF is evaporated under vacuo, and ethylacetate is added to the residue. This ethylacetate solution is successively washed with water, saturated aqueous NaHCO₃ solution and saturated aqueous NaCl solution. After drying over Na₂SO₄ and filtration, evaporation of the solvent to dryness affords hydroxamic acid derivatives in 40-60 % yield.

A substitution at position 2 of the indolic derivatives can proceed as described in Scheme 3.

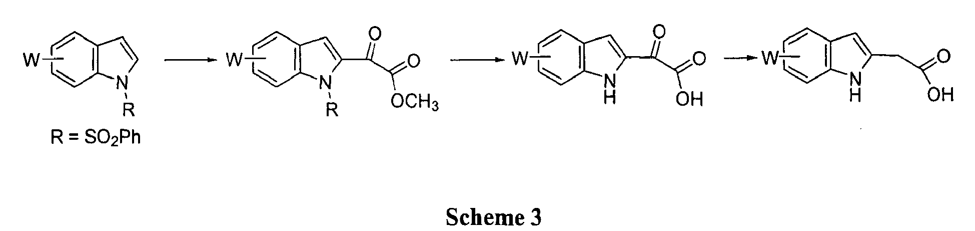

W represents a halogen, an alkyl, an aryl, an alkoxy, an aryloxy, or a thioaryl group.

Briefly, reaction 2-lithio-N-benzensulfonylindole (obtained by reaction of N-benzensulfonylindole with BuLi) with dimethyloxalate (Hasan *et al*., 1981) successively followed by basic hydrolysis and Wolf-kishner reduction affords the (indol-2-yl)acetic acid (Weller & Ford, 1984).

N-substituted indole can be prepared as described in Scheme 4.

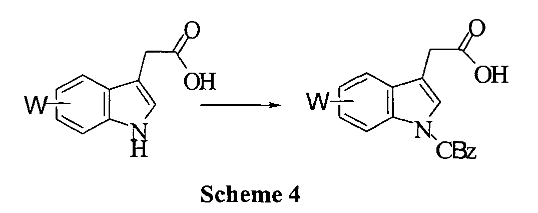

W represents a halogen, an alkyl, an aryl, an alkoxy, an aryloxy or a thioaryl group.

Briefly, N-carboxymethylation is achieved by reaction of the lithio derivative (prepared with two equiv. of lithium bis(trimethylsilyl)amide in THF at -78°C) with benzylchloroformate (Horwell *et al.,* 1997).

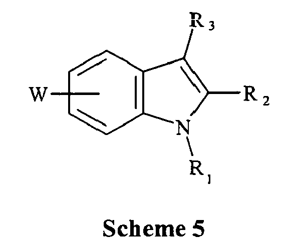

The different functionalizations of the indole nitrogen (R₁) (Scheme 5) according to the invention can be prepared as described in *Dillard et al*., (1996) and *Eissenstat et al*., (1995) for example.

The reverse hydroxamate indolic derivatives can be prepared as depicted in Scheme 6.

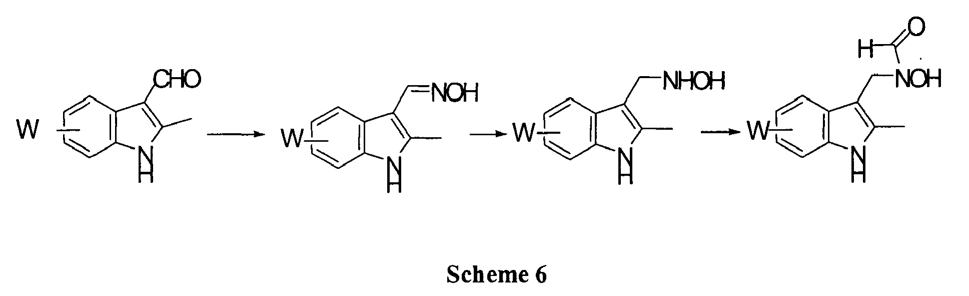

W represents a halogen, an alkyl, an aryl, an alkoxy, an aryloxy, a thioalkyl, or a thioaryl group.

Briefly, condensation of the aldehyde with hydroxylamine hydrochloride in pyridine affords the ketoxime which is further reduced under Borch's conditions (Borch *et al.,* 1971). Selective N-formylation is notably described in *Hill et al*. (2002).

The adaptations which can be brought to these procedures in order to synthesize all the compounds of formula (I) according to the invention are within the ordinary skills of the man skilled in the art.

### DESCRIPTION OF THE FIGURES

**Figure 1A, Figure 1B and Figure 1C**

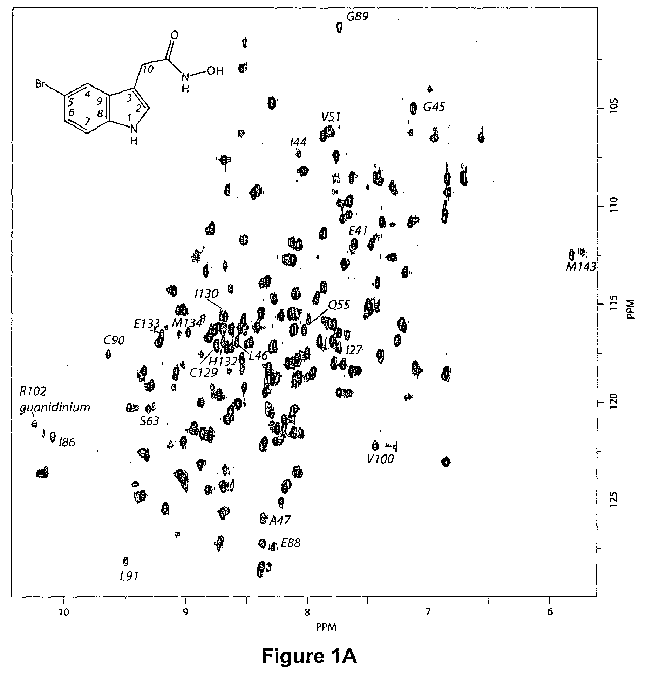

   Figure 1A: HSQC (Heteronuclear Single Quantum Correlation) footprint of 2-(5-Bromo-1H-indol-3-yl)-N-hydroxyacetamide (inset) on ¹⁵N-labelled *E. coli* peptide deformylase (1 mM). The reference spectrum is shown in black contours and the spectrum in the presence of a stoichiometric amount of the compound is overlaid in gray. Amide groups that are significantly perturbed are labeled.

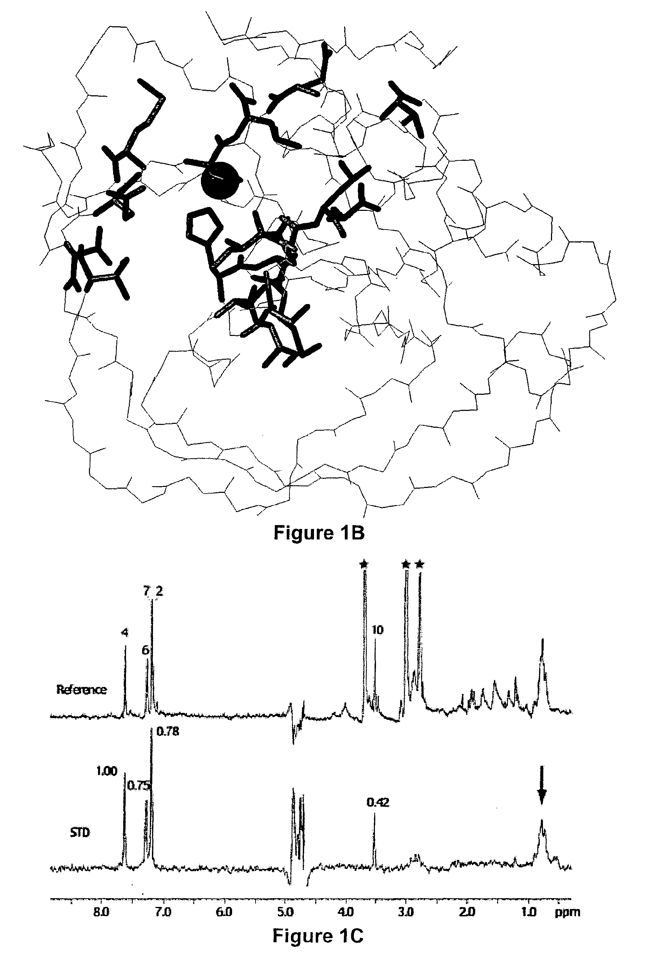

   Figure 1B: 3D structure of *E. coli* peptide deformylase. Aminoacid residues whose amide group NMR shift is perturbed by the binding of 2-(5-Bromo-1H-indol-3-yl)-*N-*hydroxyacetamide are shown in red (residues labeled in Figure 1A). The catalytic metal ion is shown as a sphere.
   Figure 1C: NMR saturation transfer difference experiment (STD) showing transfer of magnetization from *E. coli* PDF (100 µM) to 2-(5-Bromo-1H-indol-3-yl)-N-hydroxyacetamide (1 mM). Top trace: reference spectrum of the mixture. Peaks labelled with stars correspond to buffer signals. The numbered peaks corresponds to signal originating from 2-(5-Bromo-1H-indol-3-yl)-*N*-hydroxyacetamide, as labeled in Figure 1A. Bottom trace: STD signals. The arrow indicates the spectral region which was irradiated. Normalized STD intensities are indicated.
**Figure 2**

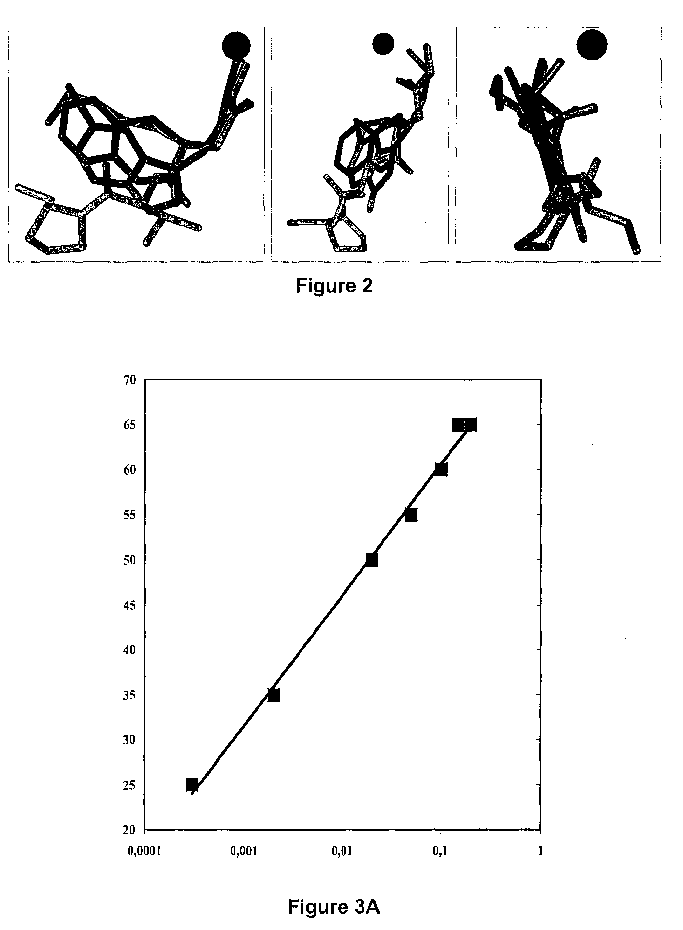

   Figure 2 represents three different views of the three dimensional model of the binding of a 2-(5-Bromo-1H-indol-3-yl)-N-hydroxyacetamide compound according to the invention to the S'₁ pocket of *Pseudomonas aeruginosa* peptide deformylase enzyme superimposed with the structures of actinonin and of a benzathiozinone-hydroxamic derivative.
**Figure 3A, Figure 3B and Figure 3C**

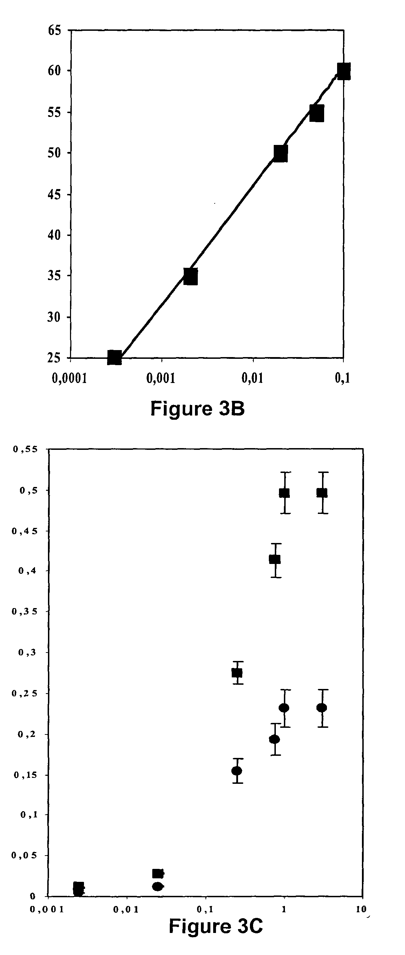

   Figures 3A and 3B respectively represent the MIC of actinonin and 2-(5-Bromo-1H-indol-3-yl)-N-hydroxyacetamide (vertical axis, µg/ml) relatively to the arabinose concentration (horizontal axis, %) for a *Escherichia coli* strain in which the *def gene* is under the control of the P_{BAD} promoter.
   Figure 3C represents the MIC of actinonin (black circles) and 2-(5-Bromo-1H-indol-3-yl)-N-hydroxyacetamide (black squares) (vertical axis, µg/ml) for a strain relatively to the xylose concentration (horizontal axis, %) for *Bacillus subilis* strains in which both the *ykrB* and *def* genes had been inactivated and in which one of the two genes was placed under the control of the P_{xylA} promoter.

### EXAMPLES

### Abbreviations used

*Bacillus subtilis,* Bs; *Escherichia coli,* Ec; *N*-formyl, Fo; Met-tRNA^{fMet} transformylase, FMT; pseudo binding constant, IC₅₀; indolic derivatives, ID; knockout, KO; minimal inhibitory concentration, MIC; metalloprotease, MP; inhibition or dissociation constant, *K*_{*d*}; matrix metalloproteases, MMPs; peptide deformylase, PDF; peptide deformylase inhibitors, PDFI; Protein Data Bank, PDB; structure activity relationship, SAR; saturation transfer difference, STD; three-dimensional, 3D.

### Example 1

### Medium-throughput PDFI screen by NMR reveal indolic derivatives as low affinity ligands of PDF

### Materials and methods

¹⁵N-labeled Ni-EcPDF1 was obtained as described (Dardel *et al.,* 1998) and dissolved in 10 mM HEPES-HCl pH 7.0 at a final concentration of 1 mM. NMR experiments were recorded on a Bruker Avance 600 MHz NMR spectrometer equipped with a 3 mm triple resonance flow-injection probe. The probe was connected to a Gilson liquid handler controlled by the NMR console (Bruker BEST® system). The injection protocol was as previously described (Tisne *et al*., 2002). For chemical shift perturbation experiments, 90 µl of 1 mM ¹⁵N-labeled EcPDF1 (PDF1B) was mixed with an equal volume of the tested ligand (3 mM), dissolved in the same buffer, in a 96-well plate and refrigerated at 4°C on a Gilson 242 Peltier rack prior to injection. For saturation transfer difference (STD, *Mayer et al*., 2001) experiments, unlabelled EcPDF1 (PDF1B) or BsPDF2 (PDF2) were used. Similar buffer and injection volumes were used except that the final protein and ligand concentrations were 20 µM and 1 mM, respectively. STD irradiation was performed by irradiating for 1 or 2 sec with the carrier set on the protein methyl massif (0.5 ppm), using a field strength γB₁/2π = 100 Hz.

### Results

The binding potency of peptide deformylase inhibitors (PDFI) with respect to PDF is essentially caused by two chemical groups:
(i) a metal-binding group and
(ii) the P1' group that binds the S1' pocket of PDF (Boularot *et al*., 2004).

The entropic increase due to the binding of either low affinity group creates potent PDFI with inhibition constants in the nanomolar range.

The S1' pocket of bacterial PDFs (PDF1Bs and PDF2s) is known to accept with low selectivity n-butyl, n-pentyl, n-hexyl, n-phenyl *(Ragusa et al*., 1999; Molteni *et al*., 2004) and other cyclic side-chains (see references in *Boularot et al*., 2004). In previous analysis, mitochondrial PDF (PDF1A) were shown to display a modified S1' pocket that cannot tolerate cyclic compounds such as phenyl derivatives for instance (Serero *et al*., 2003; *Serero et al*., 2001). In order to evidence selective PDFI (i.e. PDFI which would not target mitochondrial PDF), the approach of identifying cyclic compounds with a cyclic P1' group appeared to be the most appropriate. Therefore, the Inventors endeavored to identify cyclic compounds that would bind to bacterial PDF even with a low binding constant.

Thus, a medium-scale screen of such compounds was settled to identify such chemical groups by NMR. Using a pipeting-robot connected with a flow-injection NMR probe, a library of compounds was sequentially mixed with ¹⁵N-labeled EcPDF and injected. Chemical shift perturbations were monitored by recording HSQC-spectra which were compared to the control data obtained with the unbound-enzyme, as already described *(Meinnel et al.,* 1996; Meinnel *et al.,* 1999). Compounds, the addition of which caused resonance broadening or shifting in the {¹H-¹⁵N} HSQC protein spectrum, were identified. The affected amide groups were identified and located on the backbone of the 3D structure of EcPDF (see **Figure 1A**, for instance).

From this screen, indolic derivatives emerged as the most interesting as they showed a chemical-change pattern that corresponded to the S1' pocket of the enzyme (**Figure 1B**). Reciprocally, ligand-observe saturation transfer difference (STD) experiments (Mayer *et al*., 2001) were performed in order to obtain information on the mode of binding of these indolic derivatives on either EcPDF1 or BsPDF (**Figure 1C**).

For instance, similar results were obtained in both cases for compound 1 (see **Example** 2 below) which showed that the strongest STD effect was observed for position 4 of the indole moiety and the weakest for the methylene group attached to position 3 (**Figure 1C**). This confirmed that the indole group did bind to both PDF1B and PDF2 enzymes and suggested that the position 4 should be the most deeply buried, as it exhibited the strongest saturation transfer from the protein.

Indole and 5-bromoindole showed a binding constant in the millimolar range as probed by NMR titrations. This weak binding constant was confirmed by inhibition assays against bacterial (EcPDF1B and BsPDF2) and mitochondrial PDF (AtPDF1A) (see **Example 4** for the procedure).

In order to improve the potency of indolic derivatives (ID), the Inventors undertook the study of their structure activity relationship. Thus, substituents have been introduced on the indolyl heterocycle at positions 1 (R₁), 2 (R₂), 3 (R₃), 4 (R₄), 5 (R₅), 6 (R₆) and 7 (R₇) as labeled below:

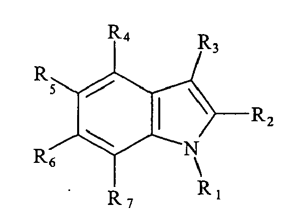

The 4, 5 and 6 positions which correspond to the P₁' (R₅) side chain were notably substituted by a lipophylic groups, such as a bromo group, to improve the fit into the S₁' pocket. At positions 2 and 3 were explored several metal binding groups linked to the indolic cylce optionally through a short CH₂ spacer. This included monodentate functions such as carboxylic acid, oxime and hydroxylamine and bidentate functions such as hydroxymethylketone, thiomethylketone and hydroxamic acid. Finally, position 1 was either free or protected by residues, such as a benzyloxycarbonyl residue, expected to mimic the P₂' and/or P₃' amino-acid groups (for a nomenclature of the various enzyme sub-sites see Boularot *et al*., 2004; *Schechter et al*., 1967).

Two such examples of indolic derivatives, carrying a hydroxamic metal chelating group a position 3 and a bromine at position 5, are presented below, as well are their inhibitory effects on PDFs and their antibiotic properties.

### Example 2

### Synthesis of compound 1: 2-(5-Bromo-1H-indol-3-yl)-N-hydroxyacetamide

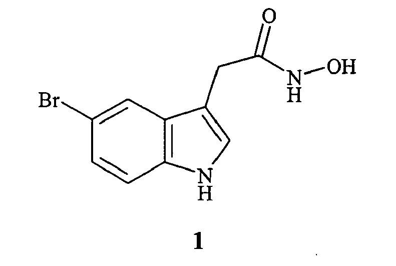

### Materials and methods

All solvents and chemicals were purchased from SDS and Aldrich, respectively. DMF, THF and CH₂Cl₂ were dried using standard procedures and stored over 4 A molecular sieves under an argon atmosphere. ¹H NMR spectra were recorded on a Bruker ARX-250 spectrometer and chemical shifts were reported in ppm downfield from TMS. IR spectra were obtained with a Perkin-Elmer Spectrum One FT-IR spectrometer equipped with a MIRacle™ single reflection horizontal ATR unit (Zirconium-Selenium crystal). FAB and CI Mass spectra were recorded at ENS in Paris. Elemental analyses were carried out by the microanalysis service at Paris VI University (France) or at Gif-sur-Yvette (CNRS, France). Experiments performed under argon were run on a vacuum line.

### Results

Starting from commercial 2-(5-bromo-1H-indol-3-yl)acetic acid (200 mg; M = 254.09, 0.787 mmoL), 116 mg of 1 was obtained in a 55% yield, according to the previously described method. Rf(C18 silica gel, ethylacetate/methanol 1/1 v:v mixture) = 0.8. Tf = 145°C. IR (cm⁻¹): 3417 (ν_{OH}) 3213 (ν_{NH}), 1633 (ν_{CO}). ¹H NMR (250 MHz, [d6] DMSO): δ 3.49 (s, 2H); 7.20-7.5 (m, 3H); 7.87 (s, 1H); 8.86 (s, 1H) ; 10.59 (s, 1H) ; 11.14 (s, 1H). CI-MS : m/z = 270 (M + H⁺). Anal.: (C₁₀H₉BrN₂O₂) C,H,N.

### Example 3

### Synthesis of compound 2: 5-bromo-3-hydroxycarbamoylmethyl-indole-1-carboxylic acid benzyl ester

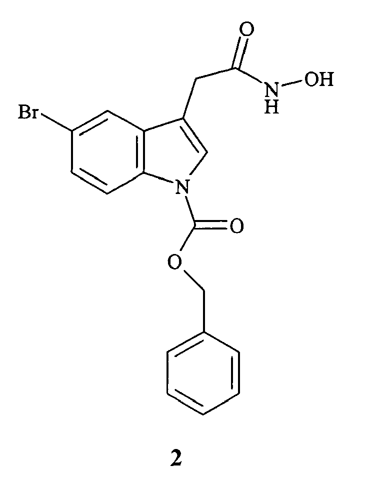

The carboxymethylation procedure described in *Horwell et al*. (1997) was followed from 2-(5-bromo-indol-3-yl)acetic acid (200mg, M =254,09, 0.787 mmoL) to give 3-carboxymethyl-5-bromo-indol-1-carboxylic acid benzyl ester (280 mg, yield: 92%). Rf (silica gel CH₂Cl₂/CH₃OH 9/1 v:v mixture) = 0.5, Tf = 88°C, IR (cm⁻¹): 1729 and 1692 (ν_{VO}). ¹H NMR (250 MHz, [d6] acetone): δ 3.80 (s, 2H); 5.51 (s, 2H); 7.24-7.82 (m, 8H); 8.12 (d, J = 8.1, 1H); 11,16 (s, 1H). CI-MS: m/z = 388 [M]. el. anal.: (C₁₈H₁₄BrNO₄).0.5 H₂O C,H,N.

Starting from 3-carboxymethyl-5-bromo-indol-1-carboxylic acid benzyl ester (120 mg, M = 388.21, 0.309 mmoL), 50 mg of 2 was obtained in a 40 % yield, according to the previously described method. ¹H NMR (250 MHz, [d6] DMSO): δ 3.43 (s, 2H); 5.52 (s, 2H); 7.50-7.60 (m, 7H); 7.91 (s, 1H); 8.07(d, J = 8.5, 1H); 8.97 (s, 1H); 10.72 (s, 1H). CI-MS: m/z = 404 [M + H⁺]. Anal.: (C₁₈H₁₅BrN₂O₄) C,H,N.

### Example 4

### In vitro assay of indolic-hydroxamic derivatives

The in *vitro* inhibition profile of compounds **1** and **2** of the invention vis-à-vis PDF of various origins was assessed and compared to that of actinonin and of RAS270 and RAS238, two synthetic inhibitors of PDF. RAS358 was synthesized as described previously (WO 02/070653). Compound RAS358 is a reverse hydroxamic compound. RAS270 corresponds to the hydroxamate derivative of RAS358.

50 mg of RAS270 was prepared in a 55 % yield, following the usual procedure for hydroxamic acid synthesis, starting from 3-tert-Butoxycarbonylamino-4-phenyl-butyric acid (commercially available) (0.308 mmoL, M = 279.33, m = 86 mg).

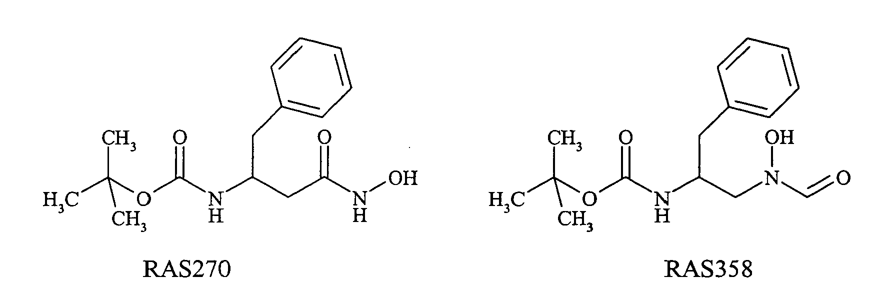

### Materials and methods

All chemicals and enzymes for protein analysis were purchased from Sigma. The peptides were from Bachem AG. Enzymes for DNA manipulation were purchased from New England Biolabs. Oligonucleotides were synthesized by MWG-AG Biotech

*Escherichia coli* PDF (EcPDF1B) and *Bacillus stearothermophilus* PDF2 (BsPDF2) were used as the representative of either bacterial PDF class and purified as described in the presence of nickel ions (Ragusa *et al.,* 1998). *Arabidospis thaliana* mitochondrial PDF (AtPDF1A) and human mitochondrial PDF (HsPDF1) were purified as described (Serero *et al*., 2001; Serero *et al.,* 2003). An assay coupling PDF activity and formate dehydrogenase activity to assess PDF activity was used. The absorbance at 340 nm of NADH (ε_{M}=6,300 M .cm⁻¹) was monitored at 37°C essentially as previously described (Lazennec *et al.,* 1997). The reaction was started by adding 5-15 µl of purified enzyme. In each case, the kinetic parameters were derived from iterative non-linear least square fits of the Michaelis-Menten equation, using the experimental data (Dardel *et al*., 1994) as previously described (Serero *et al*., 2001). All inhibitors were diluted in dimethyl sulfoxide and the final assay buffer contained 10 % of this solvent.

### Results

The results are presented in the following **Table 1**:

| **Compound** | **Actinonin** | **1** | **2** | **RAS270** | **RAS358** |
|---|---|---|---|---|---|
| ***in vitro* assay** | | | **IC**_{**50**} **(nM)**^{***a***} | | |
| EcPDF1B | 4 (TB) | 35 | 26 | 44 | 50 |
| BsPDF2 | <24 (TB) | <21 (TB) | <27 (TB) | 300 | 400 |
| AtPDF1A | <27 (TB) | 111000 | 14000 | 83000 | 150000 |
| HsPDF1m^{*b*} | <100 (TB) | 360000 | ND | ND | ND |
| HsPDF1 | <100 (TB) | 120000 | ND | ND | ND |

| | | | | | |
|---|---|---|---|---|---|
| ^{*a*} TB is tight binding *i.e*. the associated IC₅₀ measured corresponded to half of the enzyme concentration put in the assay. This concentration was identical for each enzyme type and corresponded to 40, 20 25, 600 and 13.000 nM for EcPDF1, BsPDF2, AtPDF1, HsPDFlm and HsPDF1, respectively. | | | | | |
| ^{*b*} HsPDF1m which corresponds to variant E91L/C43G of HsPDF1 was described in Serero *et al.* (2003). Similar results were obtained with the wild-type enzyme but the sensitivity was reduced as this enzyme is poorly active. This is in contrast with the double mutant which is two orders of magnitude more active. ND not determined | | | | | |

The IC₅₀ values of 1 and 2 with respect to PDF1B are approximately one order of magnitude greater than that of Actinonin and of the same order of magnitude than RAS270 and RAS358. For PDF2, the IC50 values of 1 and 2 are of the same order than that of actinonin and are more than one order of magnitude lower than that of RAS270 and RAS358. As for the mitochondrial PDFs, whereas actinonin demonstrates a potent inhibitory effect, the inhibitory effects of 1 and 2 are particularly low, so that the compounds can be said to be devoid of any inhibitory activity.

Further, it should be noted that the fact that 1 shows slow-binding mode to both PDF types, is indicative of an inhibitor induced-fit conformational change of the enzyme.

### Example 5

### In vivo assay of indolic-hydroxamic derivatives

The in *vivo* inhibition profiles of compounds 1 and 2 of the invention were assessed and compared to that of actinonin.

### Materials and methods

All bacterial strains were grown in Luria-Bertani (LB) medium (1% bacto-tryptone, 0.5 % yeast extract, 0.5% NaCl).

*Strains.* Two wild-type or pseudo wild-type *B. subtilis* strains were used, MO3482, a prototrophic skin prophage-curated derivative of JH642 (a kind gift of P. Stragier, IBPC, France) and 168 *(trpC2)* (Anagnostopoulos *et al.,* 1961). Strains MHY101 (*ΔykrB*::nm, *trpC2)* MHD101 (*Δdef*::nm, *trpC2)* were derived from 168 and previously described (Haas *et al.,* 2001). Two further *B. subtilis* strains in which both deformylase wild-type loci *(def* and *ykrB)* are deleted and one defomylase version is conditionally expressed under the control of xylose were also used (Haas *et al*., 2001): MHY103 (Δ*def*::nm; Δ*ykrB*::erm; Δ*thrC*::*xylR*-P_{xylA}-*ykrB-spc, trpC2)* and MHD103 (*Δdef*::erm; *ΔykrB*::nm; *ΔthrC*::*xylR*-P_{xylA}*-def-spc, trpC2).* 138 and its derivatives were a kind gift of C. Freiberg (Bayer AG, Germany). *E. coli* strains JM101Tr (*galk, rpsL, recA56, srl*-300::Tn*10*) and CAG1284 (λ⁻, *tolC210*::Tn*10*, *rph-)* have been described elsewhere *(Hirel et al.,* 1988; *Singer et al.,* 1989).

*E. coli CAG1284 susceptibility tests.* A susceptibility test was designed to determine the susceptibility to drugs of the *tolC* strain CAG1284. The EcPDF1 open reading frame was cloned into the pBAD vector (Invitrogen) with its C-terminus in frame with the 6-His tag. This cloning renders the synthesis of EcPDF1 dependent on arabinose concentration (Guzman *et al*., 1995). The CAG1284 strain, which is highly susceptible to several antibiotics including chloramphenicol (Sulavik *et al*., 2001), was first transformed with the pBAD construct and selected on LB medium supplemented with 50 µg/ml ampicillin, 3.4 µg/ml chloramphenicol and 0.5% glucose. Bacteria were cultured overnight in this medium at 37°C and the culture was then diluted 1:100 and used to inoculate 3 ml of medium. When the OD₆₀₀ reached 0.9, the suspension was diluted appropriately and 2 x 10⁴ of bacteria in 100 µl were layered on 30 ml of solid LB supplemented with 50 µg/ml ampicillin, actinonin (0.1-3 µM) and either glucose (0.5%) or arabinose (0.0002-0.2%) in Petri dishes. The minimum inhibitory concentration was defined as the lowest concentration of actinonin causing no growth after 18 h of incubation at 37 °C.

### Results

### 1. Inhibition of Gram-negative bacteria

Two *E. coli* derivatives (JM101Tr and CAG1284) were first used to get an insight on the minimal inhibitory concentration (MIC) values of compound 1 and to compare them with that of the natural potent PDFI actinonin *(Chen et al.,* 2000) and with that of RAS270.

With a WT strain (JM101Tr), MIC of both actinonin and 1 were similar and in the range of 30-40 µg/ml, whereas the MIC of RAS270 was above 300 µg/ml (**Tabl**e **2**).

As actinonin is strongly detoxified by the AcrAB-tolC efflux pump, a *tolC* mutant (strain CAG1284) was used to check whether this could be the same with 1. It was observed that the MIC of 1 was slightly reduced by a factor of only 5.5, in contrast to that of actinonin which was reduced 300-fold (**Table 2**). This indicates that the PDF inhibitors according to the invention are poorly detoxified by the bacterial efflux system.

**Table 2: E. coli MIC**

| **Compound** | **Actinonin** | **1** | **RAS270** |
|---|---|---|---|
| ***in vivo* assay** | **MIC (µg/ml)** | | |
| *E. coli*-JM101Tr | 27 | 40 | >300 |
| *E. coli*-CAG1284 | 0.1 | 7 | 32 |

MIC was also measured in a *tolC* context in which the expression of the *def* gene was under the control of the P_{BAD} promoter. Thus, the impact of increased PDF concentration on MIC was checked thanks to this promoter being induced by increasing arabinose concentrations *(Guzman et al*., 1995; Chen *et al*., 2000). In the case of actinonin, there is an almost linear increase of MIC with actinonin concentration (**Figure 3A**) which is indicative that (i) PDF is the primary target of actinonin, and (ii) PDF concentration is the main factor limiting the MIC in the absence of an efflux pump. When 1 was challenged in the same genetic background, a two-order MIC curve was observed (**Figure 3B**). The first part of the curve was linear which indicated that PDF was the target of 1. A saturation curve was observed above 65 µg/ml. This value is similar to that of the MIC of the WT which indicates that membrane permeability is the limiting step of the action of 1.

### 2. Inhibition of Gram-positive bacteria

In a second series of experiments, a Gram-positive model organism which expresses both a PDF1B and PDF2, *B. subtilis,* was tested. Again the MIC of actinonin was compared to that of **1** and **2**.

In a WT context (strains 168 and MO3482) the MIC of 1 and 2 (20 µg/ml) was one order of magnitude higher than that of actinonin, whereas, as could be expected from their low binding potency against PDF2, both phenyl derivatives RAS270 and RAS358 had no antibiotic properties against *B. subilis* (**Table 3**).

Then, the impact of **1** and **2** on two *B. subtilis* strain derivatives (MHD101 and MHY101) that expressed either PDF was also studied (**Table 3**).

It was found that the MIC of actinonin, 1 and 2 followed the same trend, with a reduced value in the ykrB gene-inactivated context. This confirmed that the *def gene* product was less expressed than *ykrB* (Haas *et al*., 2001) and suggested that 1 and 2 targeted indeed both PDF1B and PDF2.

**Table 3: B. subtilis MIC**

| **Compound** | **Actinonin** | **1** | **2** | **RAS270** | **RAS358** |
|---|---|---|---|---|---|
| ***in vivo* assay** | | | **MIC (µg/ml)** | | |
| *B. subtilis-168* | 1.2 | 20 | 20 | >300 | >300 |
| *B. subtilis-M03482* | 1 | 5.4 | 16 | >300 | >300 |
| *B. subtilis*-MHD101^{*c*} | 1.2 | 2.7 | 12 | >300 | >300 |
| *B. subtilis*-MHY101^{*c*} | 0.2 | 1.6 | 6 | 8 | 9 |

| | | | | | |
|---|---|---|---|---|---|
| ^{*c*} MHD is deficient in PDF1 (*def* gene) and MHY is deficient in PDF2 (*ykrB* gene) as described in Haas *et al*., (2001). | | | | | |

To prove that 1 and 2 targeted specifically PDFs in *B. subilis,* strains in which both the *ykrB* and *def* genes had been inactivated and in which one of the two gene was placed under the control of the XylR promoter, P_{xylA}, were used. Under these conditions, PDF1B or PDF2 expression is dependent of L-xylose (Haas *et al*., 2001). It was observed that the MIC of both actinonin and **1** depended upon L-xylose concentration in either MHD103 or MYD103 strain (**Figure 3C**).

Finally, the appearance of resistance to 1 and actinonin in *B. subilis* were determined and compared. A similar value (5.10⁻⁷ vs 6.10⁻⁷) was obtained, in keeping with previous measurements of others and the by-pass of the formylation-deformylation pathway induced by tranformylase gene inactivation in *Bacillus spp* (reviewed in Giglione *et al*., 2001). This data was another strong argument indicating that the target of 1 was PDF.

### Example 6

### Three-dimensional modeling of the binding of a hydroxamic indolic derivative to a PDF

### Materials and methods

Three-dimensional (3D) modeling was achieved using InsightII software (Accelrys). The 3D structures of *Pseudomonas aeruginosa* PDF (PaPDF1) bound to a benzathiozinone-hydroxamic derivative (BTH) (PDB entry 1S17, *Molteni et al*., 2004) and PaPDF1 bound to actinonin (PDB entry 1LRY, Guilloteau *et al*., 2002) were aligned. Compound 1 was constructed with the Sketcher module, aligned on the structure of both actinonin and compound BTH using the hydroxamate group as a fixed anchor and used to replace either compound in the 3D structure. The 1 structure docked to PaPDF1 was further minimized with the CharmM forcefield and the lowest energy structure selected.

### Results

The 3D modeling of compound 1 in the active site of PDF1 revealed that it fitted perfectly the S₁' pocket with the bromide group mimicking the C₄ methyl group of n-butyl or methionine side-chain, *i. e.* the optimal side-chain accepted in the S1' pocket (**Figure 2**). Furthermore, this model placed position 4 of the indole moiety deep inside the S1' pocket, buried under the side chain of His¹³², in keeping with the STD data discussed in **Example 1.**

Together, these data indicated that indolic derivatives should display antibiotic activity but not inhibit animal mitochondrial PDFs.

### Example 7

### Growth inhibition of Arabidopsis thaliana

*Arabidopsis thaliana* (ecotype WS4) growth inhibition is observed at concentrations higher than 50 µM for **1** or **2.** Besides, AtPDF1B IC₅₀ value is similar to that observed with EcPDF1B in the case of compounds 1 and **2.** Details of the experimental procedure are given in *Serero et al*. (2001) and Giglione *et al*. (2003).

Thus, the Inventors have demonstrated, through two examples **(1** and **2)** that indolic compounds according to the invention:
(i) target specifically PDF1 and PDF2, notably in Gram-negative and Gram-positive bacteria,
(ii) show essentially no inhibitory activity towards mitochondrial PDFs.

### REFERENCES

**Anagnostopoulos, C., and I. P. Crawford.** 1961. Transformation studies on the linkage of markers in the tryptophan pathway in *Bacillus subtilis.* Proc. Natl. Acad. Sci. U.S.A. **47**:378-90.
**Borch, R. F., M. D. Bernstein, and H. Dupont Durst.** 1971. The cyanohydridoborate anion as a selective reducing agent. J. Am. Chem. Soc. 93:2897-2904.3.
**Boularot, A., C. Giglione, I. Artaud, and T. Meinnel.** 2004. Structure-activity relationship and therapeutic potential of peptide deformylase inhibitors. Curr. Opin. Invest. Drugs **5**:809-822.4.
**Bracchi-Ricard, V., K. T. Nguyen, Y. Zhou, P. T. Rajagopalan, D. Chakrabarti, and D. Pei.** 2001. Characterization of an eukaryotic peptide deformylase from *Plasmodium falciparum*. Arch. Biochem. Biophys. **396:**162-170.
**Chen, D. Z., D. V. Patel, C. J. Hackbarth, W. Wang, G. Dreyer, D. C. Young, P. S. Margolis, C. Wu, Z. J. Ni, J. Trias, R. J. White, and Z. Yuan.** 2000. Actinonin, a naturally occurring antibacterial agent, is a potent deformylase inhibitor. Biochemistry **39:**1256-1262.
**Chu, M., R. Mierza, L. He, L. Xu, F. Gentile, J. Taerracciano, M. Patel, L. Miesel, S. Bohanon, C. Kravec, C. Cramer, T. O. Fischman, A. Hruza, L. Ramanatan, P. Shipkova, and T.-M. Chan.** 2001. Isolation and structure elucidation of two novel deformylase inhibitors produced by *Streptomyces* sp. Tetrahedron Lett. **42**:3549-3451.
Dardel, F. 1994. MC-Fit: Using Monte-Carlo methods to get accurate confidence limits on enzyme parameters. Comput. Appl. Biosci. **10**:273-275.
**Dardel, F., S. Ragusa, C. Lazennec, S. Blanquet, and T. Meinnel.** 1998. Solution structure of nickel-peptide deformylase. J. Mol. Biol. **280:**501-513.
**Dillard, R. D., N. J. Bach, S. E. Draheim, D. R. Berry, D. G. Carlson, N. Y. Chirgadze, D. K. Clawson, L. W. Hartley, L. M. Johnson, N. D. Jones, E. R. McKinney, E. D. Mihelich, J. L. Olkowski, R. W. Schevitz, A. C. Smith, D. W. Snyder, C. D. Sommers, and J. P.** Wery. 1996. Indole inhibitors of human nonpancreatic secretory phospholipase A2. 1. Indole-3-acetamides. J. Med. Chem. **39**:5119-36.
**Dirk, L. M., M. A. Williams, and R. L. Houtz.** 2001. Eukaryotic peptide deformylases. Nuclear-encoded and chloroplast- targeted enzymes in *Arabidopsis.* Plant Physiol. **127**:97-107.
**Eissenstat, M. A., M. R. Bell, T. E. D'Ambra, E. J. Alexander, S. J. Daum, J. H. Ackerman, M. D. Gruett, V. Kumar, K. G. Estep, E. M. Olefirowicz, and et al.** 1995. Aminoalkylindoles: structure-activity relationships of novel cannabinoid mimetics. J. Med. Chem. 38:3094-105.
**Giglione, C., and T. Meinnel.** 2001. Peptide deformylase as an emerging target for antiparasitic agents. Emerg. Therap. Targets 5:41-57.
**Giglione, C., M. Pierre, and T. Meinnel.** 2000a. Peptide deformylase as a target for new generation, broad spectrum antimicrobial agents. Mol. Microbiol. 36:1197-1205.
**Giglione, C., A. Serero, M. Pierre, B. Boisson, and T. Meinnel.** 2000b. Identification of eukaryotic peptide deformylases reveals universality of N-terminal protein processing mechanisms. EMBO J. 19:5916-5929.
**Giglione, C., O. Vallon, and T. Meinnel.** 2003. Control of protein life-span by N-terminal methionine excision. EMBO J. 22:13-23.
**Giglione, C., A. Boularot, and T. Meinnel.** 2004. Protein N-terminal methionine excision. Cell. Mol. Life Sci. 61:1455-1474.
**Gordon, J. J., B. K. Kelly, and G. A. Miller.** 1962. Actinonin: an antibiotic substance produced by an actinomycete. Nature 195:701-702.
**Grujic, M., and M. Renko.** 2002. Aminopeptidase inhibitors bestatin and actinonin inhibit cell proliferation of myeloma cells predominantly by intracellular interactions. Cancer Lett 182:113-9.
**Guilloteau, J. P., M. Mathieu, C. Giglione, V. Blanc, A. Dupuy, M. Chevrier, P. Gil, A. Famechon, T. Meinnel, and V. Mikol.** 2002. The crystal structures of four peptide deformylases bound to the antibiotic actinonin reveal two distinct types: a platform for the structure-based design of antibacterial agents. J. Mol. Biol. 320:951-962.
**Guzman, L. M., D. Belin, M. J. Carson, and J. Beckwith.** 1995. Tight regulation, modulation, and high-level expression by vectors containing the arabinose PBAD promoter. J. Bacteriol. 177:4121-4130.
**Haas, M., D. Beyer, R. Gahlmann, and C. Freiberg.** 2001. YkrB is the main peptide deformylase in *Bacillus subtilis,* a eubacterium containing two functional peptide deformylases. Microbiology 147:1783-1791.
**Hasan, I., E. R. Marinelli, L. C. Chang Lin, F. W. Fowler, and A. B. Levy.** 1981.Synthesis and reactions of *N*-protected 2-lithiated pyrroles and indoles. The *tert*-Butoxycarbonyl substituent as a protecting group. J. Org. Chem. 46:157-164.
**Hill, D. R., C. N. Hsiao, R. Kurukulasuriya, and S. J. Wittenberger.** 2002. 2,2,2-trifluoroethyl formate: a versatile and selective reagent for the formylation of alcohols, amines, and N-hydroxylamines. Org. Lett. 4:111-3.
**Hirel, P.-H., F. Lévêque, P. Mellot, F. Dardel, M. Panvert, Y. Mecbulam, and G. Fayat.** 1988. Genetic engineering of methionyl-tRNA synthetase: *in vitro* regeneration of an active synthetase by proteolytic cleavage of a methionyl-tRNA synthetase-βgalactosidase chimeric protein. Biochimie **70**:773-782.
**Horwell, D. C., D. Naylor, and H.M.G. Wilems.** 1997. 2,3-disubstituted 2-azanorbornanes as polar β-turn mimetics. Bioorg. Med. Chem. Lett. **7**: 31-36.
**Jiang, B., and X. H. Gu.** 2000. Syntheses and cytotoxicity evaluation of bis(indolyl)thiazole, bis(indolyl)pyrazinone and bis(indolyl)pyrazine: analogues of cytotoxic marine bis(indole) alkaloid. Bioorg. Med. Chem. Lett. **8**:363-71.
**Lazennec, C., and T. Meinnel.** 1997. Formate dehydrogenase-coupled spectrophotometric assay of peptide deformylase. Anal. Biochem. **244:**180-182.
**Leclerc, V., E. Fourmaintraux, P. Depreux, D. Lesieur, P. Morgan, H. E. Howell, P. Renard, D. H. Caignard, B. Pfeiffer, P. Delagrange, B. Guardiola-Lemaitre, and J. Andrieux.** 1998. Synthesis and structure-activity relationships of novel naphthalenic and bioisosteric related amidic derivatives as melatonin receptor ligands. Bioorg. Med. Chem. **6**:1875-87.
**Lee, M. D., C. Antczak, Y. Li, F. M. Sirotnak, W. G. Bornmann, and D. A. Scheinberg.** 2003. A new human peptide deformylase inhibitable by actinonin. Biochem. Biophys. Res. Commun. **312:**309-15.
**Lee MD, She Y, Soskis MJ, Borella CP, Gardner JR, Hayes PA, Dy BM, Heaney ML, Philips MR, Bornmann WG, Sirotnak FM, Scheinberg DA** (2004) Human mitochondrial peptide deformylase, a new anticancer target of actinonin-based antibiotics. J Clin Invest **114**: 1107-1116
**Lefevre-Groboillot, D., S. Dijols, J. L. Boucher, J. P. Mahy, R. Ricoux, A. Desbois, J. L. Zimmermann, and D. Mansuy.** 2001. N-hydroxyguanidines as new heme ligands: UV-visible, EPR, and resonance Raman studies of the interaction of various compounds bearing a C=NOH function with microperoxidase-8. Biochemistry **40:**9909-17.
Mayer, M., and B. Meyer. 2001. Group epitope mapping by saturation transfer difference NMR to identify segments of a ligand in direct contact with a protein receptor. J. Am. Chem. Soc. **123:**6108-17.
**Mazel, D., S. Pochet, and P. Marliere.** 1994. Genetic characterization of polypeptide deformylase, a distinctive enzyme of eubacterial translation. EMBO J. 13:914-923.
**Meinnel, T.** 2000. Peptide deformylase of eukaryotic protists: a target for new antiparasitic agents? Parasitol. Today **16**:165-168.
**Meinnel, T., S. Blanquet, and F. Dardel.** 1996, A new subclass of the zinc metalloproteases superfamily revealed by the solution structure of peptide deformylase. J. Mol. Biol. **262:**375-386.
**Meinnel, T., L. Patiny, S. Ragusa, and S. Blanquet.** 1999. Design and synthesis of substrate analogue inhibitors of peptide deformylase. Biochemistry **38**:4287-4295.
**Miesel, L., J. Greene, and T. A. Black.** 2003. Genetic strategies for antibacterial drug discovery. Nat Rev Genet **4**:442-56.
**Molteni, V., X. He, J. Nabakka, K. Yang, A. Kreusch, P. Gordon, B. Bursulaya, I. Warner, T. Shin, T. Biorac, N. S. Ryder, R. Goldberg, J. Doughty,** and **Y. He.** 2004. Identification of novel potent bicyclic peptide deformylase inhibitors. Bioorg. Med. Chem. Lett. **14:**1477-81.
**Ragusa, S., S. Blanquet,** and **T. Meinnel.** 1998. Control of peptide deformylase activity by metal cations. J. Mol. Biol. **280**:515-523.
**Ragusa, S., P. Mouchet, C. Lazennec, V. Dive, and T. Meinnel.** 1999. Substrate recognition and selectivity of peptide deformylase. Similarities and differences with metzincins and thermolysin. J. Mol. Biol. **289:**1445-1457.
**Salim, M., and A. Capretta.** 2000. Intramolecular carbenoid insertions: the reaction of α-diazoketones derived from pyrrolyl and indolyl carboxylic acids with rhodium(II) acetate. Tetrahedron **56**:8063-8069.41.
**Schechter, I., and A. Berger.** 1967. On the size of the active site in proteases. I. Papain. Bochem. Biophys. Res. Commun. **27**:157-162.
**Serero, A., C. Giglione, and T. Meinnel.** 2001. Distinctive features of the two classes of eukaryotic peptide deformylases. J. Mol. Biol **314**:695-708.
**Serero, A., C. Giglione, A. Sardini, J. Martinez Sanz, and T. Meinnel.** 2003. An unusual peptide deformylase features in the human mitochondrial N-terminal methionine excision pathway. J. Biol. Chem. **278:**52953-52963.
**Singer, M., T. A. Baker, G. Schnitzler, S. M. Deischel, M. Goel, W. Dove, K. J. Jaacks, A. D. Grossman, J. W. Erickson, and C. A.** Gross. 1989. A collection of strains containing genetically linked alternating antibiotic resistance elements for genetic mapping of *Escherichia coli.* Microbiol. Rev. **53**:1-24.
**Sulavik, M. C., C. Houseweart, C. Cramer, N. Jiwani, N. Murgolo, J. Greene, B. DiDomenico, K. J. Shaw, G. H. Miller, R. Hare, and G. Shimer.** 2001. Antibiotic susceptibility profiles of *Escherichia coli* strains lacking multidrug efflux pump genes. Antimicrob. Agents Chemother. **45**:1126-36.
**Tisne, C., and F. Dardel.** 2002. Optimisation of a peptide library for screening specific RNA ligands by flow-injection NMR. Comb. Chem. High Throughput Screen. **5**:523-9.
**Weller, D. D., and D. W. Ford.** 1984. Synthesis of ellipticine. Tetrahedron Lett. **25**:2105-2108.
**Whittaker, M., C.D. Floyd, P. Brown,** and **A.J.H. Gearing.** 1999. Design and therapeutic application of matrix metalloproteinase inhibitors. Chem Rev. **99:** 2735-2776.
**Wiesner, J., S. Sanderbrand, E. Beck, and H. Jomaa.** 2001. Seeking new targets for antiparasitic agents. Trends Parasitol. **17**:7.
**Wissner, A.** 1979. 2-hetero substituted silylated ketene acetals: reagents for the preparation of α-functionalized methyl ketones from carboxylic acid chlorides. J. Org. Chem. **44**:4717-4622. **Xu, Y., L. T. Lai, J. L. Gabrilove, and D. A. Scheinberg.** 1998. Antitumor activity of actinonin in *vitro* and in *vivo.* Clin. Cancer Res. **4**:171-176.

## Claims

1. The use of compounds having:
**a**) the following general formula (I): 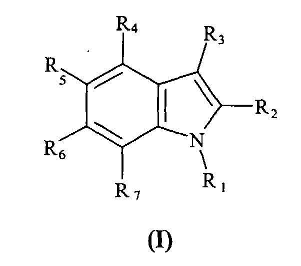 wherein:
- independently from each other, R₂ and R₃ represent H, methyl, ethyl, propyl, or a metal chelating group from 2 to 20 atoms comprising from 1 to 5 heteroatoms;
- independently from one another, R₄, R₅ and R₆ represent H, a halogen, or a group comprising from 1 to 10 carbon atoms;
- independently from each other, R₁ and R₇ represent H, or a group comprising from 1 to 50 carbon atoms;
provided that at least one of R₂ and R₃ represents a metal chelating group as defined above and at least one of R₄, R₅ and R₆ does not represent H; and
**b)** an IC₅₀ lower than about 1 µM with respect to the *Escherichia coli* nickel-bound peptide deformylase (SEQ ID NO: 1) and/or to the *Bacillus stearothermophilus* nickel-bound peptide deformylase (SEQ ID NO: 2),
or of pharmaceutically acceptable salts thereof,
for the manufacture of medicaments intended for the prevention or the treatment of bacterial or protozoan infections, or for the manufacture of herbicides.

2. The use of compounds of the general formula (I) according to claim 1, wherein, independently from each other, R₂ and R₃ represent H, methyl, ethyl, propyl, or a metal chelating group of the following formula:
-(CH₂)ₙ-X
wherein:
- n is an integer from 0 to 3, and
- X represents a group selected from the list comprising -NH₂ (amine), -NHOH (hydroxylamine), =NOH (oxime), -NHCN (cyanamide), -NH-C(NOH)NH₂ (hydroxyguanidine), -SCN (thiocyanate), -SH (thiol), -B(OH)₂ (boronic acid),
- COCH₂OH (hydroxymethylketone), -COCH₂SH (thiomethylketone), -CONHOH (hydroxamic acid), -SO₂NHOH (sulfohydroxamic acid), -NO-N=O (nonoate), -NOH-COH (reverse hydroxamic acid).

3. The use of compounds of the general formula (I) according to claim 1 or 2, wherein, independently from each other, R₂ and R₃ represent H, methyl, ethyl, propyl, or a metal chelating group of the following formula:
-(CH₂)ₙ-Y-Z
wherein:
- n is an integer from 0 to 3, and
- Y represents a group selected from the list comprising -O-, -NH-, -CHOH-, -CHF-, or 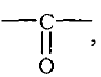 and
- Z represents a group selected from the list comprising -COOH, -CONHOH, or -COCH₂SH.

4. The use of compounds of the general formula (I) according to any of claims 1 to 3, wherein, independently from each other, R₄, R₅ and R₆ represent H, a halogen, an alkyl, an alkoxy, an acetyl, an alkoxycarbonyl, an aryl, an aryloxy, a thioalkyl, a thioaryl or an aryloxycarbonyl group, if adequate substituted by a hydroxyl, a thiol, a thioalkyl, a thioaryl, or an amino group.

5. The use of compounds of the general formula (I) according to any of claims 1 to 4, wherein R₁ represents
- H, or
- a peptide sequence, or
- an alkyl, an alkoxy, an acetyl, an alkoxycarbonyl, an aryl, an aryloxy, a thioalkyl, a thioaryl an aryloxycarbonyl, an alkoxycarbonylalkyl, or an aryloxycarbonylalkyl group, if adequate substituted by a pyridine, a morpholine, a thiomorpholoine, a piperidine, a piperazine, a hydroxyl, a thiol, a thioalkyl, a thioaryl, or an amino group.

6. The use of compounds of the general formula (I) according to any of claims 1 to 5, wherein R₇ represents H or an alkyl group comprising from 1 to 10 carbon atoms.

7. The use of compounds of the general formula (I) according to any of claims 1 to 6, wherein:
- one of R₂ and R₃ represents H or CH₃, and
- two of R₄, R₅ and R₆ represent H.

8. The use of compounds of the general formula (I) according to any of claims 1 to 7, wherein:
- R₂ represents H or CH₃, and
- R₄ and R₆ represent H, and
- R₇ represents H.

9. The use of compounds of the general formula (I) according to any of claims 1 to 8, **characterized in that** said compounds correspond to the following formulae:

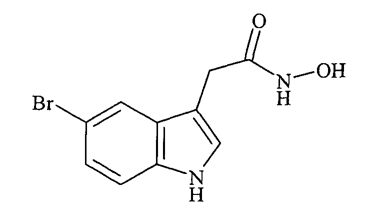

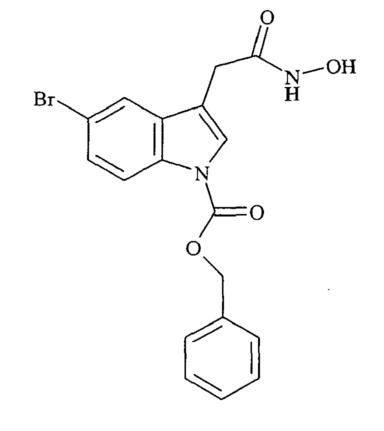

10. The use of compounds of the general formula (I) according to any of claims 1 to 9, wherein the protozoans are chosen among the apicoplast or kinetoplast bearing protozoans, and in particular are selected from the group comprising the protozoans of the following genii: *Plasmodium, Toxoplasma, Sarcocystis, Cryptosporidium, Eimera, Theileria, Babesia, Trypanosoma, Leishmania.*

11. The use of compounds of the general formula (I) according to any of claims 1 to 9, wherein the bacteria are selected from the group comprising the bacteria of the following genii: *Staphylococcus, Streptococcus, Bacillus, Enterococcus, Pseudomonas, Acinetobacter, Haemophilus, Enterobacter.*

12. A pharmaceutical composition, **characterized in that** it comprises at least one compound according to any of claims I to 9, or a pharmaceutically acceptable salt thereof, as active substance, in association with a pharmaceutically acceptable vehicle.

13. An herbicide composition, **characterized in that** it comprises at least one compound according to any of claims 1 to 9 as active substance.

14. A compound of the general formula (I), such as defined in any of claims 1 to 9.

15. A method for screening compounds comprising an indole structure intended for the prevention or the treatment of bacterial or protozoan infections, or for their use as herbicides, **characterized in that** it comprises the steps of contacting a peptide deformylase enzyme with the compounds to screen and measuring the inhibiting activity of said compounds towards said peptide deformylase enzyme.
